(19) Europäisches Patentamt
European Patent Office
Office européen des brevets

(11) Veröffentlichungsnummer: **0 078 235**
**A2**

# (12) EUROPÄISCHE PATENTANMELDUNG

(21) Anmeldenummer: 82810430.7

(22) Anmeldetag: 18.10.82

(51) Int. Cl.³: C 07 J 7/00
A 61 K 31/57

(30) Priorität: 23.10.81 CH 6790/81

(43) Veröffentlichungstag der Anmeldung: 04.05.83 Patentblatt 83/18

(84) Benannte Vertragsstaaten: AT BE CH DE FR IT LI LU NL SE

(71) Anmelder: CIBA-GEIGY AG
Patentabteilung Postfach
CH-4002 Basel(CH)

(72) Erfinder: Wieland, Peter, Dr.
Benkenstrasse 45
CH-4104 Oberwil(CH)

## (54) Dihalogenierte Steroide.

(57) Neue dihalogenierte Steroide der Formel

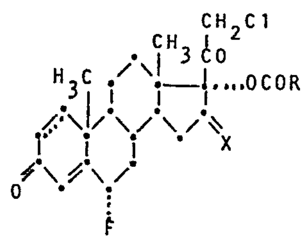

(A),

worin X Methylen oder ein α-orientiertes Methyl zusammen mit einem Wasserstoffatom und R ein Alkyl mit höchstens 6 Kohlenstoffatomen bedeutet, und ihre 1,2-Dehydroderivate, welche durch konventionelle Verfahren hergestellt werden können, weisen eine hohe entzündungshemmende Wirkung auf und sind als Antiinflammatorika verwendbar. Zudem dienen sie auch als wertvolle Zwischenprodukte zur Herstellung von antiinflammatorisch hockwirksamen polyhalogenierten Corticosteroiden.

EP 0 078 235 A2

Croydon Printing Company Ltd

0078235

4- 13610 /+

Dihalogenierte Steroide

Die vorliegende Erfindung betrifft neue dihalogenierte Steroide der Formel

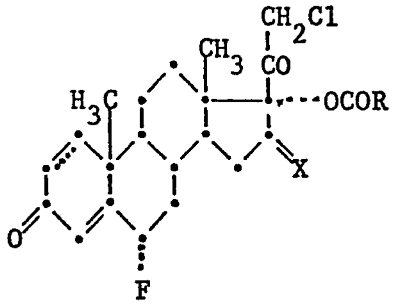

(A),

worin X Methylen oder ein α-orientiertes Methyl zusammen mit einem Wasserstoffatom und R ein Alkyl mit höchstens 6 Kohlenstoffatomen bedeutet, sowie die entsprechenden 1,2-Dehydroderivate, in welchen die punktierte Linie in der 1,2-Stellung eine zusätzliche C-C-Bindung bezeichnet, und pharmazeutische Präparate enthaltend diese Steroide, sowie Verfahren zur Herstellung derselben.

Der durch das Symbol X charakterisierte Rest ist vorzugsweise Methylen.

Das durch das Symbol R charakterisierte Alkyl kann verzweigt sein, wie Isopropyl, Isobutyl oder 1,1-Dimethylethyl, vorzugsweise ist es aber ein geradkettiges Alkyl, wie Methyl, Propyl, Butyl, Pentyl oder Hexyl und vor allem Ethyl.

Zur Herstellung von komplizierten polysubstituierten Steroidverbindungen aus einfachen Rohstoffen oder Zwischenprodukten der industriellen Produktion benötigt man einen vielstufigen Syntheseweg, in welchem jede einzelne funktionelle Gruppe separat eingeführt wird, wobei oft eine bereits eingeführte Gruppe später gegen eine unerwünschte weitere Umwandlung vorübergehend geschützt werden muss. Die Reihenfolge der einzelnen Syntheseschritte, die meistens als Analogieverfahren an sich bekannt sind, ist oft für die Oekonomie der gesamten Synthese von entscheidender Bedeutung. Bei der Synthesestrategie, d.h. bei der Auswahl der Verfahrensvarianten und ihrer Reihenfolge, wird im allgemeinen besonders darauf geachtet, dass man die Anzahl der notwendigen Syntheseschritte auf ein Minimum reduziert; dazu gehört auch die naheliegende Ueberlegung, dass eine weniger komplizierte Struktur in der Regel auch weniger Syntheseschritte zur Herstellung benötigt. Demzufolge setzt sich neuerdings gegen die frühere Tendenz, die Wirksamkeit von Grundverbindungen durch zusätzliche Substituenten weiter zu steigern, immer mehr eine alternative Forschungsstrategie durch, nämlich durch gezielte Suche die Struktur eines bestehenden komplizierten Wirkstoffs unter Beibehaltung seiner hohen Wirksamkeit zu vereinfachen. - Die erfindungsgemässen Verbindungen der Formel A sind das Resultat eines derartigen Vorgehens, indem sie einerseits antiinflammatorisch hoch wirksam, andererseits jedoch, dank ihrer einfachen Struktur, durch weniger aufwendige Verfahren herstellbar sind.

Die neuen Verbindungen der Formel A zeichnen sich durch wertvolle pharmakologische Eigenschaften, vor allem eine hervorragende antiinflammatorische Wirksamkeit aus, wie man sie z.B. bei lokaler Anwendung als Hemmung entzündlicher Vorgänge feststellen kann, sowie durch eine auffallend grosse Dissoziation zwischen antiinflammatorischem Effekt und atrophogenen Nebenwirkungen.
Sie weisen z.B. im Rattenohr-Dermatitis-Hemmtest nach Tonelli eine starke antiinflammatorische Wirkung im Dosisbereich von 10-250 µg/ml auf. Das 21-Chlor-6α-fluor-17α-hydroxy-16-methylen-pregn-4-en-3,20-dion-17-propionat besitzt z.B. eine $ED_{50}$ (wirksame Dosis, die eine 50%ige Hemmung der Symptome bewirkt) von 25 µg/ml, und das 21-Chlor-6α-fluor-17α-hydroxy-16-methylen-pregna-1,4-dien-3,20-dion-17-propionat eine $ED_{50}$ von 10 µg/ml auf. Analoge 16α-Methyl-Verbindungen zeigen Aktivitäten derselben Grössenordnung.

Die antiinflammatorische Wirkung der neuen Verbindungen kann auch im Granulomtest nachgewiesen werden, wo sie in Dosierungen von weniger als 1 µg pro Wattebausch-Pressling eine signifikante Hemmung der Granulombildung zeigen. Bis zu 500 µg pro Watte-Pressling sind in diesem impregnativen Fremdkörpergranulom-Test keine negativen Wirkungen auf den Thymus, die Nebennieren und die Körpergewichts-Entwicklung festzustellen.

Dank dieser Eigenschaften sind Verbindungen der Formel A in Indikationen, bei denen die klassischen Glucocorticoide als Antiinflammatorika mit Erfolg verwendet werden, insbesondere als Topika, z.B. zur Behandlung von entzündlichen Dermatosen, wie Ekzemen und Dermatiden, oder partiell corticostereoidresistenten Dermatosen, z.B. Psoriasis, verwendbar.

Zudem sind die Verbindungen der Formel A wertvolle Zwischenprodukte zur Herstellung anderer nützlicher Stoffe, insbesondere anderer pharmakologisch wirksamer Steroide. In dieser Beziehung haben sie z.B. eine Schlüsselstellung bei der Synthese von antiinflammatorisch hochwirksamen, topisch anwendbaren Corticosteroiden, wie von 21-Chlor-6α-fluor-9α-halogen-11β-hydroxy-16α-methyl-17α-propionyloxy-pregna-1,4-dien-3,20-dionen und Analogen davon gemäss der Britischen Patentschrift 1,537,136. Zur Ueberführung in diese wertvollen Therapeutica benötigt man lediglich konventionelle Verfahren , wie die Einführung von 11β-Hydroxyl und 9-Chlor oder 9-Fluor.

Unter den erfindungsgemässen Verbindungen der Formel A sind die 1,2-ungesättigten Derivate bevorzugt.

Unter den erfindungsgemässen Verbindungen der Formel A sind die 17-Propionate bevorzugt.

Als besonders bevorzugte Verbindungen der Formel A sind zu nennen: 21-Chlor-6α-fluor-17α-hydroxy-16-methylen-pregn-4-en-3,20-dion-17-propionat und 21-Chlor-6α-fluor-17α-hydroxy-16-methylen-pregna-1,4-dien-3,20-dion-17-propionat, sowie 21-Chlor-6α-fluor-17α-hydroxy-16α-methyl-pregn-4-en-3,20-dion-17-propionat und 21-Chlor-6α-fluor-17α-hydroxy-16α-methyl-pregna-1,4-dien-3,20-dion-17-propionat.

Die neuen Verbindungen der Formel A können erfindungsgemäss in an sich bekannter Weise durch konventionelle Verfahren der Steroid-Chemie hergestellt werden.

Die erfindungsgemässen neuen Verbindungen der Formel A werden z.B. hergestellt, indem man in einer Verbindung der allgemeinen Formel

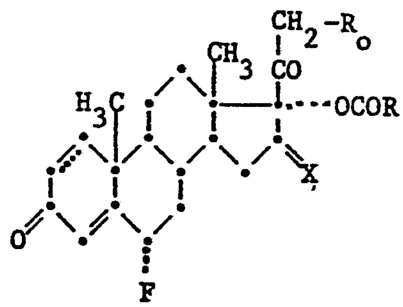

(I),

worin R, X und die punktierte Linie in der 1,2-Stellung die obgenannte Bedeutung haben und $R_o$ eine in das Chloratom überführbare Gruppe darstellt, die Gruppe $R_o$ in das Chloratom überführt, und, wenn erwünscht, in einem erhaltenen 1,2-gesättigten Endstoff die 1,2-Doppelbindung einführt.

Eine in das Chloratom überführbare Gruppe $R_o$ ist z.B. die Gruppe $-O-R_a$, worin $R_a$ für den Acylrest einer organischen Sulfonsäure steht; der Austausch der Sulfonyloxygruppe $-O-R_a$ gegen das Chloratom erfolgt in an sich bekannter Weise. Der Acylrest $R_a$ einer organischen Sulfonsäure ist insbesondere derjenige einer aliphatischen oder carbocyclischen, gegebenenfalls aromatischen Sulfonsäure. Solche Säuren sind u.a. gegebenenfalls substituierte, z.B. halogenierte, Niederalkansulfonsäuren, Cycloalkansulfonsäuren, worin der Cycloalkylrest mono- oder polycyclisch sein kann, oder gegebenenfalls durch Niederalkyl, z.B. Methyl, Niederalkoxy, z.B. Methoxy, Halogen, z.B. Chlor oder Brom, und/oder Nitro, substituierte Benzolsulfonsäuren. Als typische Beispiele solcher Säuren seien die Trifluormethansulfonsäure, (+)-Campher-10-sulfonsäure, 4-Brombenzolsulfonsäure und 3-Nitrobenzolsulfonsäure, insbesondere p-Toluolsulfonsäure und vor allem Methansulfonsäure erwähnt.

Die Austauschreaktion wird üblicherweise so durchgeführt, dass man das Ausgangsmaterial der Formel I mit einem Alkalimetallchlorid der

Formel MCl, worin M für ein Alkalimetall steht, in Anwesenheit eines aprotischen organischen Lösungsmittels, dessen dielektrische Konstante bei 29 und höher liegt, behandelt. Als Alkalimetall M kommt vorzugsweise Lithium in Betracht. Als aprotische organische Lösungsmittel können insbesondere Diniederalkylsulfoxide, z.B. Dimethylsulfoxid, N,N-Diniederalkylamide von niederaliphatischen Carbonsäuren, z.B. N,N-Dimethylformamid oder N,N-Dimethylacetamid, Niederalkannitrile, z.B. Acetonitril, Hexaniederalkylphosphoramide, z.B. Hexamethylphosphoramid, oder auch Ketone, insbesondere aliphatische oder cycloaliphatische Ketone mit 3 bis 10 Kohlenstoffatomen, wie entsprechende Alkanone, z.B. Aceton, 2-Butanon, 2- oder 3-Pentanon, 2-Hexanon oder 4-Decanon, oder Cycloalkanone mit 5 oder 6 Ringkohlenstoffatomen, z.B. Cyclopentanon oder Cyclohexanon, oder Gemische von solchen Lösungsmitteln, verwendet werden.

Die Reaktion führt man zweckmässig zwischen Raumtemperatur und der Siedetemperatur des Reaktionsgemisches durch, wobei man mit mindestens einem Aequivalent des Alkalimetallchlorids umsetzt.

Eine weitere, in das Chloratom überführbare Gruppe $R_o$ ist eine freie Hydroxylgruppe, welche gegen Chlor durch Reaktion mit einem konventionellen Halogenierungsmittel, wie Thionylchlorid, Phosphortrichlorid oder Phosphoroxychlorid, ausgetauscht werden kann, wobei man üblicherweise mit überschüssigem Halogenierungsmittel und in Anwesenheit einer organischen Base, wie insbesondere Pyridin, und unter Verdünnung mit einem inerten organischen Lösungsmittel, wie einem halogenierten Niederalkan, vornehmlich Methylenchlorid oder Chloroform, bei einer Temperatur unterhalb 80°C umsetzt. Eine bevorzugte Variante besteht darin, dass man den entsprechenden Ausgangsstoff der Formel I, worin $R_o$ für die freie Hydroxylgruppe steht, mit einem Phosphin, wie einem Triniederalkylphosphin oder vorzugsweise Triphenylphosphin, und Tetrachlorkohlenstoff behandelt. Dabei arbeitet man vorzugsweise in einem dipolaren aprotischen Medium, z.B. in Gegenwart eines entsprechenden Lösungsmittels vom Amidtyp, wie N,N-Dimethylformamid, N,N-Dimethylacetamid, 1-Methyl-2-pyrrolidon oder Hexamethylphosphoramid, unter Kühlen oder Erwärmen, z.B. in einem Temperaturbereich von etwa 10°C bis etwa 125°C, und, falls notwendig, unter erhöhtem Druck und/oder unter einer Inertgasatmosphäre.

Eine weitere gegen Chlor austauschbare Gruppe ist ein Rest, den die Gruppe $R_o$ zusammen mit der Gruppe -OCOR bildet, d.h. die Gruppierung eines von einer Niederalkancarbonsäure RCOOH abgeleiteten 17,21-Orthoesters der Partialformel

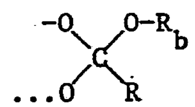 ,   (Ia)

worin R die obgenannte Bedeutung hat und $R_b$ Niederalkyl, z.B. Methyl oder Ethyl, darstellt. Der Austausch gegen Chlor in 21-Stellung unter Bildung der veresterten Hydroxylgruppe -OCOR in 17α-Stellung erfolgt in an sich bekannter Weise, z.B. durch Behandlung mit einem Chlor-übertragenden Mittel, wie einem Tri-hydrocarbyl-silylchlorid (z.B. einem gegebenenfalls substituierten Triphenylsilylchlorid, insbesondere Triphenylsilylchlorid oder Tri-p-tolylsilylchlorid, oder vorzugsweise einem Triniederalkylsilylchlorid, insbesondere Trimethylsilylchlorid) oder Triphenylmethylchlorid in organischen Lösungsmitteln, in welchen die beiden Reaktionskomponenten löslich sind und welches gegen das Halogenierungsmittel inert ist. Als solche kommen insbesondere aliphatische und cyclische Ether (wie 1,2-Dimethoxyethan, 2,5,7-Trioxanonan, Tetrahydrofuran und Dioxan) und vor allem halogenierte aliphatische Kohlenwasserstoffe (wie Tetrachlorkohlenstoff, Chloroform, Dichlorethan und insbesondere Methylenchlorid), sowie ihre zweckmässigen Kombinationen, in Betracht. Die Umsetzung erfolgt vorzugsweise bei der Siedetemperatur des Reaktionsgemisches, z.B. einer Lösung in Methylenchlorid, vornehmlich unter wasserfreien Bedingungen und gewünschtenfalls in einer inerten Atmosphäre, z.B. unter Stickstoff oder Argon.

Die Ausgangsstoffe der Formel I sind bekannt oder können nach an sich bekannter Weise hergestellt werden, z.B. Verbindungen der Formel I, worin $R_o$ für organisches Sulfonyloxy steht, durch Behandlung eines entsprechenden 6α-Fluor-17α,21-dihydroxy-16-X-pregn-4-en-3,20-dion-17-monoesters oder eines entsprechenden 1-Dehydro-Derivats mit freier 21-Hydroxylgruppe mit einem reaktionsfähigen Derivat einer organischen Sulfonsäure der Formel $R_o$-OH, insbesondere mit einem entsprechenden Sulfonsäurechlorid der Formel $R_o$-Cl, in Gegenwart einer Base, z.B. Pyridin. 17,21-Orthoester der Formel I, worin $R_o$ und -OCOR zusammen eine Gruppe der Formel Ia bilden, entstehen z.B. durch Behandeln einer 6α-Fluor-17α,21-dihydroxy-16-X-pregn-4-en-3,20-dion-Verbindung oder eines entsprechenden 1-Dehydro Derivats mit einem geeigneten Ortho-Niederalkancarbonsäureester (z.B. Ortho-Niederalkancarbonsäuretriethylester, wie vor allem Orthopropionsäuretriethylester), in Gegenwart einer starken Säure, z.B. p-Toluolsulfonsäure, und vorteilhafterweise unter Entfernen des freigesetzten Alkohols, z.B. durch azeotrope Destillation.

Die neuen Verbindungen der Formel A können ebenfalls erhalten werden, wenn man in einer entsprechenden 6β-Fluor-Verbindung der Formel

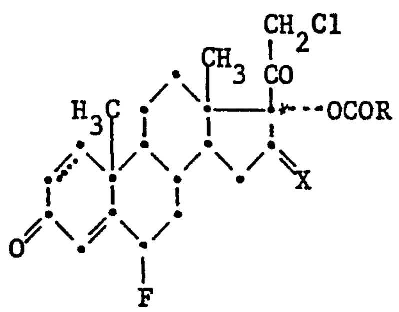

(II),

worin R, X und die punktierte Linie in der 1,2-Stellung die obgenannte Bedeutung haben, das 6β-Fluoratom zum 6α-Fluoratom epimerisiert, und gewünschtenfalls in einem erhaltenen 1,2-gesättigten Endstoff die 1,2-Doppelbindung einführt.

Die Epimerisierung erfolgt in an sich bekannter Weise, vornehmlich als säurekatalysierte Isomerisierung, indem man die Verbindung der Formel II mit einer katalytischen Menge einer starken Säure in einem inerten organischen Lösungsmittel behandelt. Als Säure verwendet man entweder anorganische Säuren, z.B. Schwefelsäure, Perchlorsäure oder eine Halogenwasserstoffsäure, wie insbesondere Chlorwasserstoff oder Bromwasserstoff, oder starke organische Säuren, z.B. Sulfonsäuren, wie insbesondere p-Toluolsulfonsäure. Als Lösungsmittel kommen z.B. flüssige Carbonsäuren, insbesondere niederaliphatische Monocarbonsäuren, wie Eisessig oder Propionsäure, und halogenierte Kohlenwasserstoffe, wie Chloroform und Methylenchlorid, sowie ihre Gemische, besonders in Betracht. Die Reaktionstemperatur liegt üblicherweise etwa zwischen dem Nullpunkt und der Zimmertemperatur. Vornehmlich wird in einer Chloroformlösung mit gasförmigem trockenem Chlorwasserstoff bei etwa 0° umgesetzt.

Die als Ausgangsstoff der Formel II verwendete 6β-Fluorverbindung kann auch direkt im Reaktionsgemisch in situ gebildet und sofort, ohne Isolierung, epimerisiert werden. Bei einer besonders vorteilhaften Variante des obigen Verfahrens wird anstelle der obengenannten 4,5-ungesättigten 6β-Fluorverbindung der Formel II einer ihrer möglichen Ausgangsstoffe, und zwar das 21-Chlor-6β-fluor-5α,17α-bis-(niederalkancarbonyloxy)-16-X-pregnan-3,20-dion der Formel III

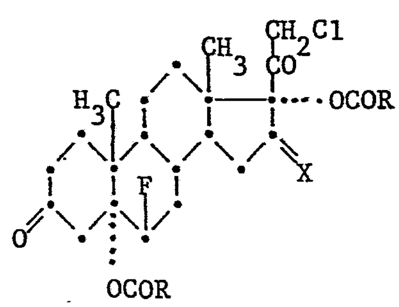

(III),

worin X und R die obgenannte Bedeutung haben, unter den oben beschriebenen Reaktionsbedingungen mit einer katalytischen Menge einer starken Säure in

einem inerten organischen Lösungsmittel, behandelt. - Gewünschtenfalls kann man aber auch zweistufig vorgehen, indem man in der ersten Stufe in der Verbindung der Formel III, unter Bildung der mit der 3-Oxogruppe konjugierten 4,5-Doppelbindung, die veresterte 5α-Hydroxylgruppe durch Behandlung mit Säure eliminiert, und in der zweiten Stufe in der erhaltenen, isolierten Verbindung der Formel II die säurekatalysierte Isomerisierung des 6β-ständigen Fluoratoms zur thermodynamisch stabileren 6α-Konfiguration durchführt. Da zur β-Eliminierung der 5-ständigen Niederalkancarbonyloxygruppe schon die geringe Acidität einer Carbonsäure genügt, sind bekanntlich zu diesem Zweck flüssige Carbonsäuren, die zugleich auch als Lösungsmittel dienen, besonders geeignet, darunter insbesondere niederaliphatische Monocarbonsäuren, wie vor allem Eisessig und Propionsäure. Vorzugsweise wird dann bei einer erhöhten Temperatur von etwa 50° bis zur Siedetemperatur des Reaktionsgemisches in Abwesenheit einer stärkeren Säure gearbeitet.

In 1,2-Stellung gesättigte Ausgangsstoffe der Formel II können in an sich bekannter Weise auch auf einem alternativen Weg hergestellt werden, indem man eine entsprechende, in 6-Stellung unsubstituierte 21-Chlor-17α-niederalkancarbonyloxy-16-X-pregn-4-en-3,20-dion-Verbindung durch Behandlung mit einem Niederalkylorthoformiat (z.B. insbesondere Ethyl- oder Methyl-orthoformiat) unter Säurekatalyse zum entsprechenden 3-Enolether (d.h. 3-Niederalkoxy-3,5-dien) umwandelt und diesen mit Perchlorylfluorid in an sich bekannter Weise umsetzt.

Diejenigen Ausgangsstoffe der Formel III, worin X für Methylen steht, sind besonders vorteilhaft aus entsprechenden 21-Chlor-6β-fluor-3β,5α,17α-trihydroxy-16-methylen-pregnan-20-on-3-Niederalkanoyl-5,17-bis(niederalkancarbonyl)-estern, die in der Europäischen Patentanmeldung 81810152.9 beschrieben sind, erhältlich. Dabei wird im 21-Chlor-6β-fluor-3β,5α,17α-trihydroxy-16-methylen-pregnan-20-on-3-niederalkanoyl-5,17-diniederalkancarbonylester der Formel IV

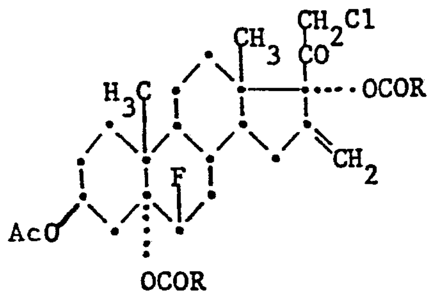

(IV),

worin R die obgenannte Bedeutung hat und Ac ein Niederalkanoyl darstellt, zunächst die veresterte 3β-Hydroxylgruppe solvolytisch freigesetzt (womit der entsprechende 5,17-Diniederalkancarbonylester entsteht), und anschliessend zur 3-Oxogruppe oxidiert. Die selektive solvolytische Freisetzung der sekundären 3β-Hydroxylgruppe in Gegenwart von analog veresterten tertiären Hydroxylgruppen in den Stellungen 5α- und 17α- wird in an sich bekannter Weise unter Säurekatalyse durchgeführt, z.B. in einem Niederalkanol, wie Methanol, Ethanol oder Isopropylalkohol in Gegenwart einer Mineralsäure, wie Chlorwasserstoffsäure oder Schwefelsäure. Falls die 3-Hydroxylgruppe im Ausgangsstoff der Formel (IV) als Formiat vorliegt, ist die selektive Freisetzung besonders glatt und kann auch mit schwach basischen Mitteln, z.B. mit einem Aequivalent Alkalimetallhydrocarbonat, wie Natrium- oder Kaliumhydrogencarbonat, bei Raumtemperatur erfolgen. Auch die anschliessende Oxidation (Dehydrierung) der freien 3β-Hydroxylgruppe zur Oxogruppe unter Bildung des 21-Chlor-6β-fluor-5α,17α-diniederalkancarbonyloxy-16-methylen-pregnan-3,20-dions der Formel III ($X = CH_2$) geschieht in der konventionellen, allgemein bekannten Weise, vorteilhaft z.B. mit einer Verbindung des 6-wertigen Chroms (wie Chromtrioxid oder Chromsäure und ihre Alkalimetallsalze), wobei man als Reaktionsmedium Niederalkancarbonsäuren, wie Essig- oder Propionsäure, oder ein Keton, wie Aceton, gegebenenfalls unter Verdünnung mit einem halogenierten Niederalkan, wie Methylenchlorid oder Chloroform, verwendet und die Reaktionstemperatur vorzugsweise unterhalb der Raumtemperatur hält. Eine bevorzugte Variante ist die Oxidation mit einer Lösung von Chromtrioxid in wässriger Schwefelsäure (Jones-Reagens), die man

üblicherweise in Aceton bei einer Temperatur zwischen etwa -10° bis etwa 25°, vorzugsweise in der Umgebung des Nullpunktes, durchführt.

Der oben erwähnte Rest Ac stellt einen Niederalkanoylrest mit 1-7 Kohlenstoffatomen dar, z.B. einen solchen, der sich vom weiter oben definierten Alkylrest R ableitet, besonders aber den Acetyl- und Formylrest. (Zur Unterscheidung davon wird in der ganzen Beschreibung der ähnliche Rest R.CO-, welcher jedoch gemäss der eingangs angegebenen Definition 2-7 Kohlenstoffatome enthält, als der Niederalkancarbonylrest bezeichnet).

Die neuen Verbindungen der Formel A können ebenfalls erhalten werden, indem man in einer entsprechenden 17α-Hydroxy-Verbindung der Formel V

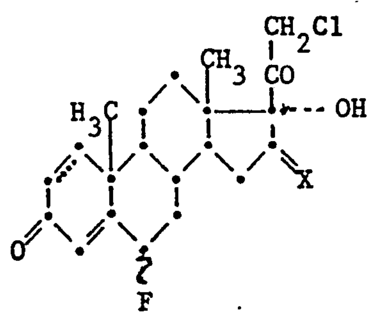

(V),

worin X und die punktierte Linie in der 1,2-Stellung die obgenannte Bedeutung haben und das 6-Fluoratom α- oder β-orientiert sein kann, die freie 17α-Hydroxylgruppe mit dem oben definierten Rest RCO- einer Niederalkancarbonsäure verestert und zugleich ein gegebenenfalls vorhandenes 6β-Fluoratom zum 6α-Fluoratom epimerisiert, und gewünschtenfalls in einem erhaltenen 1,2-gesättigten Endstoff die 1,2-Doppelbindung einführt. Zu dieser Umwandlung verwendet man an sich bekannte, herkömmliche Methoden zur Veresterung von schwierig veresterbaren tertiären Hydroxylgruppen, z.B. Behandlung der Verbindung der Formel V, die eine solche Hydroxylgruppe in der 17α-Stellung hat, mit einem symmetrischen Anhydrid einer geeigneten Niederalkancarbonsäure, z.B. mit Propionsäureanhydrid, unter Katalyse

mittels einer starken Mineralsäure, wie insbesondere der Perchlorsäure, oder einer organischen Sulfonsäure, wie p-Toluolsulfonsäure. Vornehmlich verwendet man jedoch als Veresterungsmittel ein reaktionsfähiges gemisches Anhydrid der entsprechenden Niederalkancarbonsäure, insbesondere ein solches mit Trifluoressigsäure, z.B. das gemischte Propionsäure-Trifluoressigsäure-Anhydrid. Die Umsetzung erfolgt üblicherweise bei Zimmertemperatur unter Ausschluss von Wasser in einem inerten organischen Lösungsmittel, wie einem gegebenenfalls halogenierten Kohlenwasserstoff, z.B. Benzol, Toluol, Cyclohexan, bzw. Chloroform oder Methylenchlorid, oder einem Ether, wie Diethylether, Dioxan oder Tetrahydrofuran, mit überschüssigem Veresterungsmittel. Dieses wird vorteilhaft unmittelbar vor der Umsetzung im Reaktionsgemisch vorbereitet, indem man die entsprechende Niederalkancarbonsäure mit einer etwa äquivalenten Menge Trifluoracetanhydrid, gegebenenfalls unter Kühlung, vermischt und 30-60 Minuten bei Zimmertemperatur reagieren lässt.

Ausgangsstoffe der Formel V kann man in an sich bekannter Weise herstellen. So sind z.B. Verbindungen der Formel V, worin X für Methylen steht, durch die säurekatalysierte Isomerisierung des entsprechenden 21-Chlor-16α,17α-epoxy-(6α- oder 6β)-fluor-16β-methyl-pregn-(4-en oder 1,4-dien)-3,20-dions der nachstehend definierten Formel VI erhältlich.

Die neuen Verbindungen der Formel A, worin X für Methylen steht, können auch erhalten werden, indem man in einem entsprechenden 16β-Methyl-16α,17α-epoxid der Formel VI

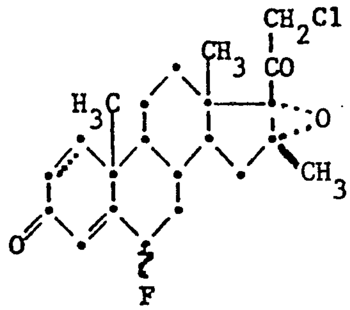

(VI),

worin die punktierte Linie in 1,2-Stellung die zusätzliche C-C-Bindung des 1,2-Dehydroderivats bezeichnet und das 6-Fluoratom α- oder β-orientiert sein kann, die 16-Methyl-16,17-epoxy-Gruppierung einer säurekatalysierten acylierenden Isomerisierung mit Ringöffnung (und gegebenenfalls zugleich der Epimerisierung des 6β-Fluoratoms) unterwirft und gewünschtenfalls in einem erhaltenen 1,2-gesättigten Endstoff die 1,2-Doppelbindung einführt.

Durch die acylierende Epoxidringöffnung wird in einem Verfahrensschritt die 16β-Methyl-16α,17α-epoxy-Gruppierung direkt zur gewünschten 16-Methylen-17α-niederalkancarbonyloxy-Gruppierung umgewandelt. Die Umwandlung wird in an sich bekannter Weise durchgeführt, indem man die Epoxidverbindung der Formel VI mit einem von einer Niederalkancarbonsäure RCOOH abgeleiteten Acylierungsmittel in einem wasserfreien Medium in Gegenwart eines stark sauren Katalysators umsetzt. Als Acylierungsmittel wird vorzugsweise ein reaktionsfähiges Derivat der Niederalkancarbonsäure, wie ein Anhydrid und insbesondere ein symmetrisches Anhydrid der Formel $(RCO-)_2O$, worin R die obgenannte Bedeutung hat, verwendet. Ein stark saurer Katalysator ist vorzugsweise eine sauerstoffhaltige Säure, wie Schwefelsäure, Perchlorsäure oder eine organische Sulfonsäure, z.B. p-Toluolsulfonsäure, p-Brombenzolsulfonsäure oder Benzolsulfonsäure; als Lösungsmittel kann man Niederalkancarbonsäuren, insbesondere solche, die dem Acylierungsmittel entsprechen, verwenden, ferner kann man auch in aprotischen Lösungsmitteln, z.B. Kohlenwasserstoffen, insbesondere aromatischen Kohlenwasserstoffen, wie Benzol oder Toluol, oder in halogenierten aliphatischen Kohlenwasserstoffen, wie insbesondere Chloroform und Methylenchlorid, mit Vorteil die Reaktion führen, wobei man bei Temperaturen von ca. 0° bis zu Siedehitze der Reaktionsmischung, vorzugsweise bei Zimmertemperatur, arbeitet.

Die Ausgangsstoffe der Formel VI sind nach bekannten Analogieverfahren erhältlich, z.B. durch Behandeln eines 21-Chlor-(6α- oder 6β)-fluor-16-methyl-pregna-4,16-dien-3,20-dions mit einem konventionellen Epoxidierungsmittel, vornehmlich mit einer Peroxycarbonsäure, wie Perbenzoesäure, Phthalmonoperoxysäure und insbesondere m-Chlorperbenzoesäure. Das letztgenannte Steroid-4,16-dien ist z.B. aus der entsprechenden 6-unsubstituierten Pregna-4,16-dien-3,20-dion-Verbindung durch konventionelle Einführung des Fluoratoms in die 6-Stellung (z.B. durch die Ueberführung in einen 3-Enolether, insbesondere ein 3-Niederalkoxy-3,5-dien, dessen Behandeln mit Perchlorylfluorid und gegebenenfalls säurekatalysiertes Epimerisieren des eingeführten β-orientierten 6-Fluoratoms) zugänglich.

Die neuen Verbindungen der Formel A können auch erhalten werden, indem man in einer entsprechenden 1,2-gesättigten Verbindung mit geschützter 3-Oxogruppe die Schutzgruppe unter Freisetzung der Oxogruppe entfernt und, wenn eine 1,2-Dehydroverbindung gewünscht ist, in einem erhaltenen 1,2-gesättigten Endstoff die 1,2-Doppelbindung einführt.

Als Verbindungen mit geschützter 3-Oxogruppe kommen z.B. 3-Ketale und 3-Thioketale in Betracht. Die ersteren leiten sich vorzugsweise von Niederalkanolen, wie Methanol oder Ethanol, und insbesondere von α- oder β-Glykolen, wie 1,2- oder 1,3-Propandiol, 1,2- 2,3- oder 1,3-Butandiol und vor allem Ethylenglykol, ab; Thioketale leiten sich vorzugsweise von analog gebauten Thiolen und Dithiolen ab, wobei das entsprechende 3,3-Ethylenthioketal besonders bevorzugt ist. Bei den

3-Ketalen ist die Doppelbindung üblicherweise in die 5,6-Stellung verschoben, wogegen sie bei den 3-Thioketalen in der Regel die ursprüngliche 4,5-Stellung beibehält; für das Resultat der Freisetzung der 3-Oxogruppe ist jedoch diese Lage der Doppelbindung belanglos, da diese bei der Freisetzung jeweils in die Konjugation mit der 3-Oxogruppe migriert.

Als Verbindungen mit geschützter 3-Oxogruppe kommen insbesondere 3-substituierte 3,5-Dien-Verbindungen, wie 3-Enamine, 3-Enolether und 3-Enolester, in Betracht, vor allem die Verbindungen der Formel VII

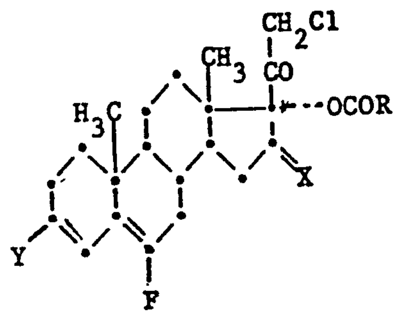

(VII),

worin R und X die obgenannte Bedeutung haben und Y eine solvolytisch abspaltbare Gruppe darstellt.

Die solvolytisch abspaltbare Gruppe Y ist z.B. eine tertiäre Aminogruppe, wie vor allem die Pyrrolidinogruppe, oder vornehmlich eine veretherte oder veresterte Hydroxylgruppe, wie insbesondere eine Niederalkoxygruppe (z.B. Methoxy oder Ethoxy) bzw. Niederalkancarbonyloxygruppe -OCOR der obgenannten Bedeutung.

Alle genannten Schutzgruppen können in an sich bekannter Weise, z.B. durch solvolytisches Abspalten, entfernt werden; üblicherweise erfolgt das Abspalten unter den allgemeinen Bedingungen der Säurekatalyse und in Gegenwart von Wasser (d.h. als säurekatalysierte Hydrolyse). Als Säurekatalyse bezeichnet man dabei eine Behandlung in Anwesenheit einer anorganischen Säure (z.B. einer sauerstoffhaltigen Säure, wie Schwefelsäure oder Perchlorsäure, oder einer Halogenwasserstoffsäure,

wie der Chlor-, Brom- oder Jodwasserstoffsäure), einer organischen Sulfonsäure (wie insbesondere der p-Toluolsulfonsäure oder Benzolsulfonsäure) oder einer stärkeren Carbonsäure (wie Trifluoressigsäure, Chloressigsäure, Oxalsäure oder Ameisensäure). In Spezialfällen, wie zur Abspaltung von labileren Schutzgruppen (z.B. von Enamin- oder Enolether-Gruppen), kann man auch schwächere Carbonsäuren (wie Benzoe- oder Essigsäure) für die Katalyse verwenden.

Die hydrolytische Abspaltung erfolgt üblicherweise in einem neutralen organischen Lösungsmittel, vorzugsweise einem solchen, welches mindestens teilweise mit Wasser mischbar ist, z.B. in einem Niederalkanol (wie insbesondere Methanol, Ethanol oder Isopropylalkohol), einem offenkettigen oder cyclischen Ether (z.B. Diethylether, 1,2-Dimethoxyethan, Dioxan oder Tetrahydrofuran), oder einem aliphatischen Keton (wie Aceton) und gegebenenfalls, insbesondere zur Erhöhung der Löslichkeit, mit Beimischung eines halogenierten Niederalkans (wie insbesondere Chloroform oder Methylenchlorid); die Reaktionstemperatur liegt zwischen etwa -10°C bis Siedetemperatur des Reaktionsgemisches, vornehmlich zwischen Nullpunkt und der Raumtemperatur.

Bei Thioketalen kann man vorzugsweise mit Zusatz einer Schwefel-bindenden Verbindung (z.B. eines Metallsalzes, wie Cadmiumcarbonat, und/oder eines Schwermetallsalzes, wie Quecksilber(II)chlorid) arbeiten. Da das letztgenannte Mittel selber in Anwesenheit von Wasser stark sauer reagiert, ist bei seiner Anwendung keine zusätzliche Säure als Katalysator notwendig.

Enolacylate der Formel VII, worin Y für Niederalkancarbonyloxy -OCOR steht, kann man vornehmlich auch durch säurekatalysierte Alkoholyse, insbesondere in einem wasserfreien Niederalkanol, wie Methanol, Ethanol oder Isopropylalkohol, in Gegenwart einer Mineralsäure (wie insbesondere Chlorwasserstoff oder Schwefelsäure), unter Erhaltung der tertiären 17α-Niederalkancarbonyloxygruppe abspalten. Die Spaltung

von Enolacylaten kann schliesslich auch durch basenkatalysierte Hydrolyse erfolgen; vornehmlich wird sie mit schwach basischen anorganischen Reagentien, z.B. mit einem Aequivalent Alkalimetallhydrogencarbonat (wie Natriumhydrogencarbonat oder Kaliumhydrogencarbonat), und unter milden Reaktionsbedingungen, z.B. bei oder unterhalb Raumtemperatur, durchgeführt.

Die als Ausgangsstoffe verwendeten Derivate mit geschützter 3-Oxogruppe, wie diejenigen der Formel VII, sind gemäss konventionellen Verfahren der Steroidchemie zugänglich, z.B. aus entsprechenden 6β-Fluorderivaten mit freier 3-Oxogruppe unter den allgemein bekannten Verfahren der Ketalisierung, Thioketalisierung, Enolacylierung und Bildung von Enolethern bzw. Enaminen. (Diese Verfahren sind allgemein durch die säurekatalysierte Behandlung der 3-Oxoverbindung mit einem entsprechenden Alkohol, Glykol, Dithiol, Säureanhydrid, Niederalkanolorthoester bzw. sekundären Amin unter Ausschluss oder Abscheidung von Wasser charakterisiert). Enolacylate der Formel VII, worin Y die oben definierte Gruppe -OCOR darstellt, können z.B. auch entstehen, wenn man die oben beschriebenen Acylierungsreaktionen der Ausgangsstoffe der Formel V oder VI unter energischen Bedingungen (z.B. erhöhter Temperatur, verlängerter Reaktionszeit und höheren Konzentrationen von Reaktionsteilnehmern und insbesondere vom Katalysator) vornimmt.

Die gewünschtenfalls durchzuführende nachträgliche Einführung der 1,2-Doppelbindung in die 1,2-gesättigten Verbindungen unter Entstehung von entsprechenden 1,2-Dehydroderivaten erfolgt in an sich bekannter Weise, z.B. durch Dehydrieren. Mann kann dazu biologische Dehydrierungsverfahren anwenden, z.B. mittels der Mikroorganismen Corynebacterium simplex oder Septomyxa affinis oder ihrer Enzymsysteme, oder mit Selendioxid in einem organischen Lösungsmittel, z.B. z.B. tert.-Butylalkohol, behandeln. Vorzugsweise lässt man jedoch 2,3-Dichlor-5,6-dicyan-1,4-benzochinon, etwa bei Siedehitze während

mehreren, z.B. 6-24 Stunden einwirken; als Lösungsmittel verwendet man übliche Lösungsmittel, z.B. aromatische Kohlenwasserstoffe, wie Benzol oder Xylol, niederaliphatische Alkohole, wie Ethanol, Propylalkohol oder tert.-Butylalkohol, niederaliphatische Ketone, wie Aceton oder 2-Butanon, aliphatische Ester, wie Ethylacetat, oder cyclische Ether, wie Dioxan oder Tetrahydrofuran.

Im oben geschilderten erfindungsgemässen Verfahren werden vorzugsweise Reaktionsmittel und Zwischenprodukte verwendet, die zu den besonders hervorgehobenen, insbesondere den spezifisch genannten, Endstoffen und Zwischenprodukten führen; die Umsetzungen werden jedoch vorzugsweise mit 1,2-gesättigten Zwischenprodukten vorgenommen und die 1,2-Doppelbindung im letzten Schritt eingeführt.

Sofern nicht spezifisch definiert wird, bezieht sich in der ganzen Beschreibung die Bezeichnung "nieder" im Zusammenhang mit einem Kohlenwasserstoffrest auf einen solchen mit höchstens 7 Kohlenstoffatomen.

Die Erfindung betrifft auch diejenigen Ausführungsformen der obigen Verfahren, bei denen man von einer auf irgendeiner Stufe als Zwischenprodukt erhältlichen Verbindung ausgeht und die fehlenden Schritte durchführt, oder bei denen ein Ausgangsstoff unter den Reaktionsbedingungen gebildet wird.

Die vorliegende Erfindung betrifft auch pharmazeutische Präparate für Menschen und Säugetiere, welche die neuen oben beschriebenen Verbindungen der Formel A in einer therapeutisch wirksamen Menge als aktive Substanz zusammen mit einem pharmazeutischen Trägermaterial enthalten, sowie ihre Herstellung. Als Träger verwendet man organische oder anorganische Stoffe, die für die enterale, insbesondere orale, und intrauterine, parenterale, oder topische Gabe geeignet sind. Für die Bildung derselben kommen solche Stoffe in Frage, die mit den neuen

Verbindungen nicht reagieren, wie z.B. Wasser, Gelatine, Milchzucker, Stärke, Magnesiumstearat, Talk, pflanzliche Oele, Benzylalkohol, Gummi, Polyalkylenglykole, Vaseline, Cholesterin und andere bekannte Arzneimittelträger. Die pharmazeutischen Präparate können in fester Form, z.B. als Tabletten, Dragées oder Kapseln, oder in flüssiger oder halbflüssiger Form als Lösungen, Suspensionen, Emulsionen, Salben oder Cremen vorliegen. Gegebenenfalls sind diese pharmazeutischen Präparate sterilisiert und/oder enthalten Hilfsstoffe, wie Konservierungs-, Stabilisierungs-, Netz- oder Emulgiermittel, Salze zur Veränderung des osmotischen Druckes oder Puffer. Sie können auch noch andere therapeutisch wertvolle oder biologisch wirksame Stoffe enthalten.

Dabei kommen in erster Linie topisch anwendbare pharmazeutische Präparate, wie Crèmen, Salben, Pasten, Schäume, Tinkturen und Lösungen, in Frage, die von etwa 0,001% bis etwa 0,5%, vorzugsweise etwa 0,005 bis etwa 0,05%, des Wirkstoffs enthalten.

Crèmen sind Oel-in-Wasser-Emulsionen, die mehr als 50% Wasser aufweisen. Als ölige Grundlage verwendet man in erster Linie Fettalkohole, z.B. Lauryl-, Cetyl- oder Stearylalkohole, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Vaseline (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Ethylenoxidaddukte davon, wie Polyglycerinfettsäureester oder Polyoxyethylensorbitan-fettsäureester (Tweens), ferner Polyoxyethylen-fettalkoholether oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B.

Cetylalkohol oder Stearylalkohol, verwendet. Zusätze von Wasserphase sind u.a. Mittel, welche die Austrocknung der Crème vermindern, z.B. Polyalkohole, wie Glycerin, Sorbit, Propylenglykol und/oder Polyethylenglykole, ferner Konservierungsmittel und Riechstoffe.

Salben sind Wasser-in-Oel-Emulsionen, die bis zu 70%, vorzugsweise jedoch von etwa 20% bis etwa 50% Wasser oder wässrige Phase enthalten. Als Fettphase kommen in erster Linie Kohlenwasserstoffe, z.B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole, bzw. Wollwachs, enthalten. Emulgatoren sind entsprechende lipophile Substanzen, wie Sorbitan-fettsäureester (Spans), z.B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/oder Polyethylenglykol, sowie Konservierungsmittel und Riechstoffe.

Fettsalben sind wasserfrei und enthalten als Grundlage insbesondere Kohlenwasserstoffe, z.B. Paraffin, Vaseline und/oder flüssige Paraffine, ferner natürliche oder partial-synthetische Fette, z.B. Kokosfettsäuretriglycerid, oder vorzugsweise gehärtete Oele, z.B. hydriertes Erdnuss- oder Rizinussöl, ferner Fettsäurepartialester des Glycerins, z.B. Glycerinmono- und -distearat, sowie z.B. die im Zusammenhang mit den Salben erwähnten, die Wasseraufnahmefähigkeit steigernden Fettalkohole, Emulgatoren und/oder Zusätze.

Pasten sind Crèmen und Salben mit sekretabsorbierenden Puderbestandteilen, wie Metalloxiden (z.B. Titandioxid oder Zinkoxid, ferner Talk und/oder Aluminiumsilikate), welche die Aufgabe haben, vorhandene Feuchtigkeit oder Sekrete zu binden.

Schäume werden aus Druckbehältern verabreicht und sind in Aerosolform

vorliegende flüssige Oel-in-Wasser-Emulsionen, wobei halogenierte Kohlenwasserstoffe, wie Chlorfluorniederalkane (z.B. Dichlordifluormethan und Dichlortetrafluorethan) als Treibmittel verwendet werden. Als Oelphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyethylen-sorbitan-fettsäureester (Tweens), und solchen mit vorwiegend lipophilen Eigenschaften, wie Sorbitanfettsäureester (Spans). Dazu kommen die üblichen Zusätze, wie Konservierungsmittel.

Tinkturen und Lösungen weisen meistens eine wässerig-ethanolische Grundlage auf, der u.a. Polyalkohole, z.B. Glycerin, Glykole, und/oder Polyethylenglykol, als Feuchthaltemittel zur Herabsetzung der Verdunstung, und rückfettende Substanzen, wie Fettsäureester mit niedrigen Polyethylenglykolen, d.h. im wässerigen Gemisch lösliche, lipophile Substanzen als Ersatz für die der Haut mit dem Ethanol entzogenen Fettsubstanzen, und, falls notwenig, andere Hilfs- und Zusatzmittel beigegeben sind.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei Verarbeitung des Wirkstoffs als Lösung wird dieser in der Regel vor der Emulgierung in einer der beiden Phasen gelöst; bei Verarbeitung als Suspension wird er nach der Emulgierung mit einem Teil der Grundlage vermischt und dann dem Rest der Formulierung beigegeben.

Die Dosierung des Wirkstoffs, z.B. der oben besonders hervorgahobenen Verbindungen, erfolgt im Prinzip analog derjenigen von anerkannten topischen Antiinflammatorika vom Corticoid-Typ; sie hängt jedoch auch

einerseits von Spezies, Körpergewicht, Alter und individuellem Zustand des Warmblüters, andererseits von der Applikationsweise ab; eine geeignete Dosis kann im Routine-Test in bekannter Weise für jeden individuellen Fall festgestellt werden.

Die Erfindung betrifft auch eine Methode zur Linderung oder Behebung von krankhaften entzündlichen Zuständen des Körpers, und insbesondere der Haut, eines Warmblüters, vor allem des Menschen, welche durch die Behandlung dieses Körpers oder Körperteils, vorzugsweise in topischer Applikation, mit einer antiinflammatorisch wirksamen Menge einer Verbindung der Formel A, allein oder in Form eines pharmazeutischen Präparats, charakterisiert ist. - Unter der Bezeichnung "eine antiinflammatorisch wirksame Menge" ist eine solche Menge des Wirkstoffs zu verstehen, die zu einer signifikanten Inhibition der Entzündung ausreicht.

In den nachfolgenden Beispielen wird die praktische Durchführung der vorliegenden Erfindung noch näher illustriert, ohne sie dadurch in ihrem Umfang einzuschränken. Die Temperaturen werden vor- und nachstehend in Celsiusgraden angegeben; wenn nicht spezifisch bezeichnet, sind Lösungsmittelgemische in Vol.% oder in Vol.:Vol-Verhältnis, Feststofflösungen in Gew.%, d.h. als Gewicht des Feststoffs (in Gramm) in 100 Volumenteilen (in ml) der Lösung angegeben.

Beispiel 1: Durch eine Lösung von 3,4 g 21-Chlor-6β-fluor-5α,17α-dihydroxy-16-methylen-pregnan-3,20-dion-dipropionat in 340 ml Chloroform wird 6 Stunden unter Eiskühlung Chlorwasserstoff geleitet. Die Lösung wird auf Wasser geleert, mit Methylenchlorid extrahiert, mit 3%-iger Natriumhydrogencarbonat-Lösung gewaschen, getrocknet und im Vakuum eingedampft. Den Rückstand chromatographiert man an 100 g Kieselgel. Mit Toluol wird zunächst ein Gemisch von 21-Chlor-6β-fluor-17α-hydroxy-16-methylen-pregn-4-en-3,20-dion-propionat und 21-Chlor-

6α-fluor-17α-hydroxy-16-methylen-pregn-4-en-3,20-dion-propionat eluiert. Anschliessend wird die reine 6α-Fluorverbindung erhalten, die nach Umlösen aus Methylenchlorid-Ether bei 175-178,5° schmilzt.

Der Ausgangsstoff kann folgendermassen hergestellt werden:

Eine Mischung von 4,7 g 21-Chlor-6β-fluor-3β,5α,17α-trihydroxy-16-methylen-pregnan-20-on-3-acetat-5,17-dipropionat und 235 ml Methanol und 31 ml einer 3,85 N-Lösung von Chlorwasserstoff in Isopropanol wird vier Stunden bei Zimmertemperatur gerührt, auf gesättigte Natriumhydrogencarbonat-Lösung gegossen und mit Methylenchlorid extrahiert. Die organischen Lösungen werden mit verdünnter Kochsalzlösung gewaschen, getrocknet und im Vakuum eingedampft.

Zur Lösung des erhaltenen rohen 21-Chlor-6β-fluor-3β,5α,17α-trihydroxy-16-methylen-pregnan-20-on-5,17-dipropionates in 32 ml Methylenchlorid und 128 ml Aceton gibt man unter Rühren und Eiskühlung 4,4 ml einer 8 N-Lösung von Chromsäure in Schwefelsäure-Wasser (Jones Reagens). Nach 30 Minuten wird mit einer Lösung von 4,1 g Natriumacetat in 130 ml Wasser versetzt und mit Methylenchlorid extrahiert. Die organischen Lösungen werden mit Natriumhydrogencarbonat-Lösung und verdünnter Kochsalzlösung gewaschen, getrocknet und im Vakuum eingedampft. Durch Kristallisation des Rückstandes aus Ether erhält man 3,57 g 21-Chlor-6β-fluor-5α,17α-dihydroxy-16-methylen-pregnan-3,20-dion-dipropionat vom Smp. 179-183°.

Beispiel 2: Eine Lösung von 500 mg 21-Chlor-6α-fluor-17α-hydroxy-16-methylen-pregn-4-en-3,20-dion-propionat und 625 mg umkristallisiertes 2,3-Dichlor-5,6-dicyan-1,4-benzochinon in 20 ml Dioxan wird während 23 Stunden unter Rückfluss gekocht. Die abgekühlte Reaktionslösung wird mit 125 ml gesättigter Natriumhydrogencarbonat-Lösung versetzt. Nach 30-minütigem Rühren wird mit Methylechlorid extrahiert,

mit verdünnter Kochsalzlösung gewaschen, getrocknet und im Vakuum eingeengt. Der Rückstand wird durch Dünnschichtchromatographie auf Kieselgel im System Toluol-Aceton-(4:1) aufgetrennt. Das erhaltene 21-Chlor-6α-fluor-17α-hydroxy-16-methylen-pregna-1,4-dien-3,20-dion-propionat schmilzt nach Umlösen aus Methylenchlorid-Ether bei 158-159,5°.

Beispiel 3: Eine Lösung von 5,2 g 6α-Fluor-17α,21-dihydroxy-16α-methyl-pregn-4-en-3,20-dion-17-propionat in 20 ml Pyridin wird bei -10° mit 2,5 ml Methansulfonylchlorid versetzt. Nach 16-stündigem Stehenlassen bei 0° wird unter Rühren auf eine Mischung von 25 ml konzentrierter Salzsäure und 200 ml Eiswasser geleert. 30 Minuten später wird abgenutscht, mit Wasser gewaschen, in Methylenchlorid gelöst, getrocknet und im Vakuum eingedampft. Der Rückstand wird mit einer Mischung von 50 ml Dimethylformamid und 10 g Lithiumchlorid während 16 Stunden bei einer Badtemperatur von 95° in Stickstoffatmosphäre verrührt. Die abgekühlte Reaktionsmischung wird auf 500 ml Wasser geleert. Nach 30-minütigem Rühren wird abgenutscht, mit Wasser gewaschen, in Methylenchlorid gelöst, getrocknet und im Vakuum eingedampft. Bei der Chromatographie des Rückstandes an 150 g Kieselgel wird das 21-Chlor-6α-fluor-17α-hydroxy-16α-methyl-pregn-4-en-3,20-dion-propionat mit Toluol-Ethylacetat (49:1) eluiert; nach Umlösen aus Methylenchlorid-Ether wird ein Produkt vom Smp. 226-227° erhalten.

Das als Ausgangsstoff verwendete 17α-Propionat kann folgendermassen erhalten werden:

Eine Mischung von 2 g 6α-Fluor-17α,21-dihydroxy-16α-methyl-pregn-4-en-3,20-dion, 10 ml Dimethylformamid, 2 ml Orthopropionsäureethylester und 100 mg p-Toluolsulfonsäure wird während 1 1/2 Stunden in Argonatmosphäre verrührt. Dann wird auf Eiswasser, enthaltend 1 ml Pyridin, geleert, abgenutscht und mit Wasser gewaschen. Den Rückstand löst man

in Chloroform, worauf vom Wasser abgetrennt, getrocknet und im Vakuum eingedampft wird. Der Rückstand wird aus Ether-Pentan umkristallisiert. Zur Lösung von 1 g des erhaltenen Kristallisates in 50 ml Alkohol gibt man eine Lösung von 278 mg Oxalsäure in 3 ml Wasser. Nach 1 1/2-stündigem Rühren bei 50° wird auf 50 ml Wasser geleert und der Alkohol bei 40° im Vakuum abgedampft. Dann wird abgenutscht, mit Wasser gewaschen, in Methylenchlorid gelöst, getrocknet und im Vakuum eingedampft. Nach Umkristallisation des Rückstandes aus Methylenchlorid-Ether schmilzt das erhaltene 6α-Fluor-17α,21-dihydroxy-16α-methyl-pregn-4-en-3,20-dion-17-propionat bei 112-122°.

Beispiel 4: Eine Mischung von 500 mg 21-Chlor-6α-fluor-17α-hydroxy-16α-methyl-pregn-4-en-3,20-dion-propionat, 20 ml Dioxan und 625 mg umkristallisiertes 2,3-Dichlor-5,6-dicyan-1,4-benzochinon wird während 22 Stunden unter Rückfluss gekocht. Die abgekühlte Reaktionslösung wird mit 125 ml gesättigter Natriumhydrogencarbonat-Lösung verrührt. Nach 30-minütigem Rühren wird mit Methylenchlorid extrahiert, mit verdünnter Kochsalzlösung gewaschen, getrocknet und im Vakuum eingedampft. Der Rückstand wird auf Kieselgel dünnschichtchromatographisch im System Toluol-Aceton (9:1) aufgetrennt. Das erhaltene 21-Chlor-6α-fluor-17α-hydroxy-16α-methyl-pregna-1,4-dien-3,20-dion-propionat schmilzt nach Umlösen aus Methylenchlorid-Ether bei 212-222°.

Beispiel 5: Eine Salbe, enthaltend 0,1% 21-Chlor-6α-fluor-17α-hydroxy-16-methylen-pregna-1,4-dien-3,20-dion-17-propionat kann wie folgt hergestellt werden:

Zusammensetzung

| | |
|---|---|
| 21-Chlor-6α-fluor-17α-hydroxy-16-methylen-pregna-1,4-dien-3,20-dion-17-propionat | 0,1% |
| Vaseline | 45,0% |
| Paraffinöl | 19,6% |

| | |
|---|---|
| Cetylalkohol | 5,0% |
| Bienenwachs | 5,0% |
| Sorbitan-sesquioleat | 5,0% |
| p-Hydroxybenzoesäureester | 0,2% |
| Riechstoff | 0,1% |
| Wasser | 20,0% |

Die Fettstoffe und Emulgatoren werden zusammengeschmolzen. Das Konservierungsmittel wird in Wasser gelöst, und die Lösung in die Fettschmelze bei erhöhter Temperatur einemulgiert. Nach dem Erkalten wird eine Suspension des Wirkstoffs in einem Teil der Fettschmelze in die Emulsion eingearbeitet und schliessend Riechstoff zugegeben.

Patentansprüche (für alle benannten Länder ausser Oesterreich)

1. Eine Verbindung der Formel

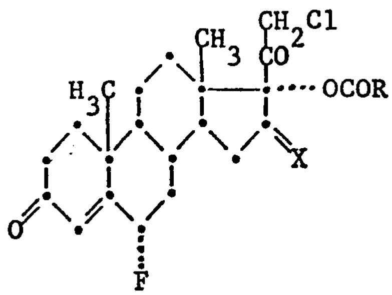 (A),

worin X Methylen oder ein α-orientiertes Methyl zusammen mit einem Wasserstoffatom, und R ein Alkyl mit höchstens 6 Kohlenstoffatomen bedeutet, und die punktierte Linie in der 1,2-Stellung eine zusätzliche Doppelbindung des entsprechenden 1,2-Dehydroderivats bezeichnet.

2. Eine 1,2-ungesättigte Verbindung gemäss Anspruch 1.

3. Eine Verbindung gemäss Anspruch 1, worin X für Methylen steht.

4. Eine Verbindung gemäss Anspruch 1, worin R für Ethyl steht.

5. Eine Verbindung gemäss Anspruch 1, die das 21-Chlor-6α-fluor-17α-hydroxy-16-methylen-pregn-4-en-3,20-dion-17-propionat ist.

6. Eine Verbindung gemäss Anspruch 1, die das 21-Chlor-6α-fluor-17α-hydroxy-16-methylen-pregna-1,4-dien-3,20-dion-17-propionat ist.

7. Eine Verbindung gemäss Anspruch 1, die das 21-Chlor-6α-fluor-17α-hydroxy-16α-methyl-pregn-4-en-3,20-dion-17-propionat ist.

8. Eine Verbindung gemäss Anspruch 1, die das 21-Chlor-6α-fluor-17α-hydroxy-16α-methyl-pregna-1,4-dien-3,20-dion-17-propionat ist.

9. Verfahren zur Herstellung einer Verbindung der Formel

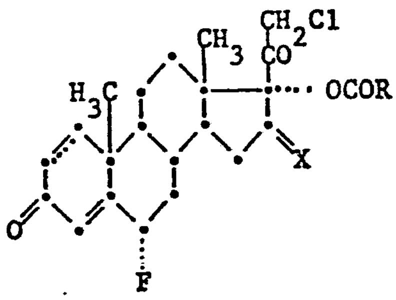

(A),

worin X Methylen oder ein α-orientiertes Methyl zusammen mit einem Wasserstoffatom und R ein Alkyl mit höchstens 6 Kohlenstoffatomen bedeutet, wobei die punktierte Linie in der 1,2-Stellung eine zusätzliche Doppelbindung eines 1,2-Dehydroderivats bezeichnet, dadurch gekennzeichnet, dass man

a) in einer Verbindung der allgemeinen Formel

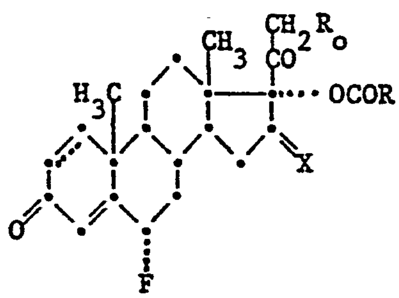

(I),

worin R, X und die punktierte Linie in der 1,2-Stellung die obgenannte Bedeutung haben und $R_o$ eine in das Chloratom überführbare Gruppe darstellt, die Gruppe $R_o$ in das Chloratom überführt, oder

b) in einer entsprechenden 6β-Fluorverbindung der Formel

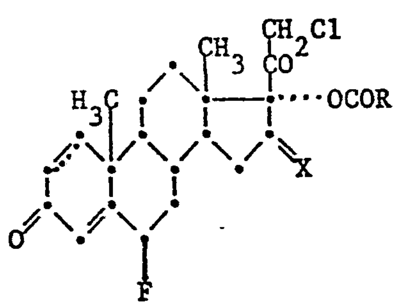

(II),

worin R, X und die punktierte Linie in der 1,2-Stellung die obgenannte Bedeutung haben, das 6β-Fluoratom zum 6α-Fluoratom epimerisiert, oder
c) in einer entsprechenden 17α-Hydroxy-Verbindung der Formel V

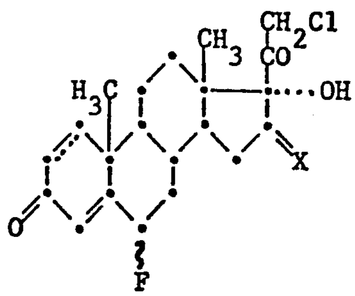

(V),

worin X und die punktierte Linie in der 1,2-Stellung die obgenannte Bedeutung haben und das 6-Fluoratom α- oder β-orientiert sein kann, die freie 17α-Hydroxylgruppe mit dem oben definierten Rest RCO- einer Niederalkancarbonsäure verestert und zugleich ein gegebenenfalls vorhandenes 6β-Fluoratom zum 6α-Fluoratom epimerisiert, oder
d) zur Herstellung einer Verbindung der Formel A, worin X für Methylen steht, in einem entsprechenden 16-Methyl-16,17-epoxid der Formel VI

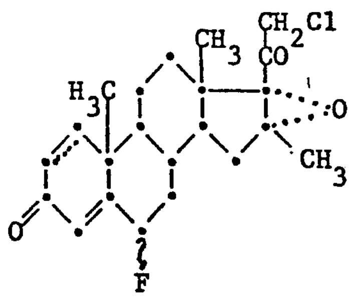

(VI),

worin die punktierte Linie in 1,2-Stellung die zusätzliche C-C-Bindung des 1,2-Dehydroderivats bezeichnet und das 6-Fluoratom α- oder β-orientiert sein kann, die 16-Methyl-16,17-epoxy-Gruppierung einer säurekatalysierten acylierenden Isomerisierung mit Ringöffnung und ein gegebenenfalls vorhandenes 6β-Fluoratom einer Epimerisierung unterwirft, oder
e) in einer Verbindung, die einer 1,2-gesättigten Verbindung der Formel A entspricht, aber die 3-Oxo-Gruppe in geschützter Form trägt,

die Schutzgruppe unter Freisetzung der Oxogruppe entfernt, oder, gewünschtenfalls,
f) zur Herstellung einer 1,2-Deyhdroverbindung in einem erhaltenen 1,2-gesättigten Endstoff die 1,2-Doppelbindung einführt.

10. Ein pharmazeutisches Präparat enthaltend eine der in den Ansprüchen 1-8 definierten Verbindungen zusammen mit einem pharmazeutischen Trägermaterial.

11. Verwendung einer Verbindung gemäss einem der Ansprüche 1-8 als antiinflammatorisches Mittel.

12. Verwendung einer Verbindung gemäss einem der Ansprüche 1-8 als Zwischenprodukt zur Synthese von 6,9,21-trihalogenierten Corticosteroiden.

Patentansprüche (für Oesterreich)

1. Verfahren zur Herstellung einer Verbindung der Formel

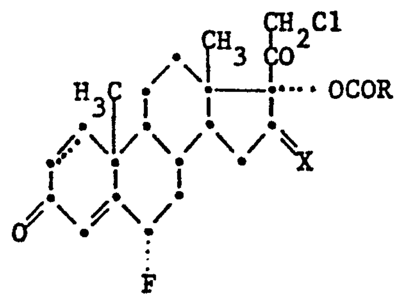

(A),

worin X Methylen oder ein α-orientiertes Methyl zusammen mit einem Wasserstoffatom und R ein Alkyl mit höchstens 6 Kohlenstoffatomen bedeutet, wobei die punktierte Linie in der 1,2-Stellung eine zusätzliche Doppelbindung eines 1,2-Dehydroderivats bezeichnet, dadurch gekennzeichnet, dass man

a) in einer Verbindung der allgemeinen Formel

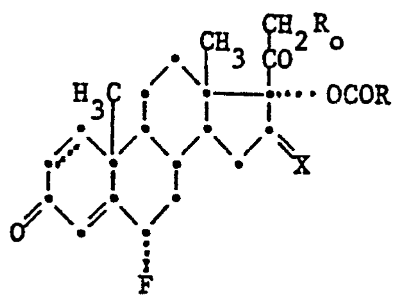

(I),

worin R, X und die punktierte Linie in der 1,2-Stellung die obgenannte Bedeutung haben und $R_o$ eine in das Chloratom überführbare Gruppe darstellt, die Gruppe $R_o$ in das Chloratom überführt, oder

b) in einer entsprechenden 6β-Fluorverbindung der Formel

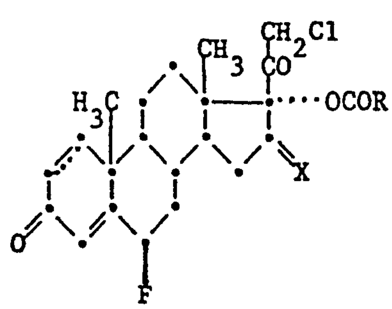

(II),

worin R, X und die punktierte Linie in der 1,2-Stellung die obgenannte Bedeutung haben, das 6β-Fluoratom zum 6α-Fluoratom epimerisiert, oder
c) in einer entsprechenden 17α-Hydroxy-Verbindung der Formel V

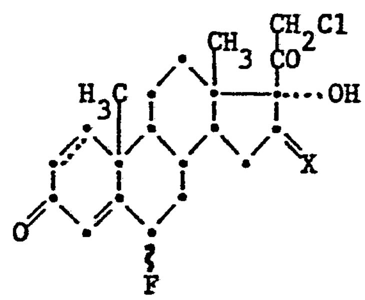

(V),

worin X und die punktierte Linie in der 1,2-Stellung die obgenannte Bedeutung haben und das 6-Fluoratom α- oder β-orientiert sein kann, die freie 17α-Hydroxylgruppe mit dem oben definierten Rest RCO- einer Niederalkancarbonsäure verestert und zugleich ein gegebenenfalls vorhandenes 6β-Fluoratom zum 6α-Fluoratom epimerisiert, oder
d) zur Herstellung einer Verbindung der Formel A, worin X für Methylen steht, in einem entsprechenden 16-Methyl-16,17-epoxid der Formel VI

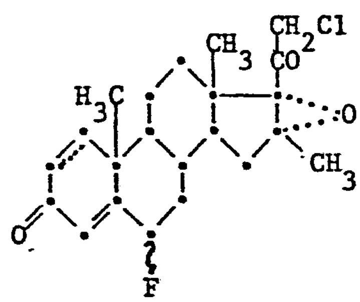

(VI),

worin die punktierte Linie in 1,2-Stellung die zusätzliche C-C-Bindung des 1,2-Dehydroderivats bezeichnet und das 6-Fluoratom α- oder β-orientiert sein kann, die 16-Methyl-16,17-epoxy-Gruppierung einer säurekatalysierten acylierenden Isomerisierung mit Ringöffnung und ein gegebenenfalls vorhandenes 6β-Fluoratom einer Epimerisierung unterwirft, oder
e) in einer Verbindung, die einer 1,2-gesättigten Verbindung der Formel A entspricht, aber die 3-Oxo-Gruppe in geschützter Form trägt,

die Schutzgruppe unter Freisetzung der Oxogruppe entfernt, oder gewünschtenfalls
f) zur Herstellung einer 1,2-Dehydroverbindung in einem erhaltenen 1,2-gesättigten Endstoff die 1,2-Doppelbindung einführt.

2. Verfahren gemäss Anspruch 1, dadurch gekennzeichnet, dass man eine 1,2-ungesättigte Verbindung der Formel A herstellt.

3. Verfahren gemäss Anspruch 1, dadurch gekennzeichnet, dass man eine Verbindung der Formel A, worin R für Ethyl steht, herstellt.

4. Verfahren gemäss Anspruch 1, dadurch gekennzeichnet, dass man eine Verbindung der Formel A, worin X Methylen bedeutet, herstellt.

5. Verfahren gemäss Anspruch 1, dadurch gekennzeichnet, dass man das 21-Chlor-6α-fluor-17α-hydroxy-16-methylen-pregn-4-en-3,20-dion-17-propionat herstellt.

6. Verfahren gemäss Anspruch 1, dadurch gekennzeichnet, dass man das 21-Chlor-6α-fluor-17α-hydroxy-16-methylen-pregna-1,4-dien-3,20-dion-17-propionat herstellt.

7. Verfahren gemäss Anspruch 1, dadurch gekennzeichnet, dass man das 21-Chlor-6α-fluor-17α-hydroxy-16α-methyl-pregn-4-en-3,20-dion-17-propionat herstellt.

8. Verfahren gemäss Anspruch 1, dadurch gekennzeichnet, dass man das 21-Chlor-6α-fluor-17α-hydroxy-16α-methyl-pregna-1,4-dien-3,20-dion-17-propionat herstellt.

9. Verfahren zur Herstellung von pharmazeutischen Präparaten enthaltend eine der in den Ansprüchen 1-8 definierten Endstoffen als Wirkstoff, dadurch gekennzeichnet, dass man mindestens einen solchen Wirkstoff mit mindestens einem pharmazeutischen Trägermaterial verarbeitet.

10. Verwendung einer, gemäss einem der Ansprüche 1-8 erhaltenen Verbindung als antiinflammatorisches Mittel oder als Zwischenprodukt zur Synthese von 6,9,21-trihalogenierten Corticosteroiden.